# EUROPÄISCHE PATENTANMELDUNG

(11) **EP 2 422 753 A1**
(43) Veröffentlichungstag der Anmeldung: **29.02.2012**
(21) Anmeldenummer: 11006804.6
(22) Anmeldetag: 20.08.2011
(51) Int. Cl.: A61F 2/44

(54) **Instrumentarium zum eindrehen eines Implantats in ein Bandscheibenfach**

(30) Priorität: 30.08.2010 DE 102010035832
(71) Anmelder: spontech spine intelligence group AG, 70182 Stuttgart (DE)
(72) Erfinder: Copf, Franz, 70184 Stuttgart (DE); Mohr, Marcus, 70839 Gerlingen (DE); Taddia, Lino, 78333 Stockach (DE); Fischer, Erik, 70619 Stuttgart (DE); Anic, Tomislav, 70599 Stuttgart (DE); Pross, Gerhard, 71039 Weil im Schönbuch (DE)
(74) Vertreter: Schwanhäusser, Gernot

(57) **Zusammenfassung**

Ein Instrumentarium zum Eindrehen eines mit einem Außengewinde (43) versehenen Implantats (42) in ein Bandscheibenfach (170) umfasst eine Führungshülse (10). Diese stützt sich mit ihrem distalen Ende während des Eindrehvorgangs an Wirbeln (W1, W2) ab, die das Bandscheibenfach (170) begrenzen. Ferner umfasst das Instrumentarium ein Eindrehinstrument (40), das einen Schaft (44) hat, der in die Führungshülse (10) einführbar ist. Der Schaft (44) hat ein distales Ende, an dem das Implantat (42) drehfest festlegbar ist, und ein proximales Ende, an dem ein Drehgriff (128, 60) drehfest befestigt ist und das während des Eindrehvorgangs über das proximale Ende der Führungshülse (10) hinausragt. Erfindungsgemäß weist das Eindrehinstrument eine Restvorschubanzeige (74, 96, 16, 18, 52, 106, 107) auf, die dem Chirurgen während des Eindrehvorgangs quantitativ anzeigt, wie weit das Implantat (42) in axialer Richtung von einer axialen Sollposition entfernt ist, die relativ zu dem Ort festgelegt ist, an dem sich die Führungshülse (10) an den Wirbeln (W1, W2) abstützt.

## Beschreibung

### HINTERGRUND DER ERFINDUNG

### 1. Gebiet der Erfindung

Die Erfindung betrifft ein Instrumentarium zum Eindrehen eines mit einem Außengewinde versehenen Implantats in ein Bandscheibenfach.

### 2. Beschreibung des Standes der Technik

Bei degenerierten Wirbelsäulensegmenten besteht eine Behandlungsmöglichkeit darin, die betroffene Bandscheibe ganz oder teilweise zu entfernen und ein Implantat in das Bandscheibenfach einzusetzen. Häufig ist das Implantat mit einem Außengewinde versehen und kann dadurch in das präparierte Bandscheibenfach eingeschraubt werden. Das Implantat hält die benachbarten Wirbel auf Abstand und ermöglicht es, dass das Bandscheibenfach nach und nach verknöchert, was schließlich zu einer Fusion der benachbarten Wirbel führt.

Ein Beispiel für ein solches Implantat ist in der WO 2005/082292 A1 beschrieben. Das bekannte Implantat hat einen abgerundeten Kopf mit einem konischen Gewindeabschnitt, einen Zentralabschnitt mit großflächigen Fenstern und eine Basisabschnitt, der Befestigungsmittel für ein Eindrehinstrument aufweist.

Bevor das Implantat eingedreht werden kann, muss das Bandscheibenfach in der Regel distrahiert werden. Hierzu kann beispielsweise ein System aus mehreren aufeinander abgestimmten Führungshülsen und Distraktoren verwendet werden, wie es in der in der noch unveröffentlichten deutschen Patentanmeldung "System zum Distrahieren eines Bandscheibenfachs" der Anmelderin beschrieben ist, die am 24.08.2010 eingereicht wurde.

Nach erfolgter Distraktion ragen zwei an den distalen Enden der Führungshülsen vorspringende Distanzzungen in das Bandscheibenfach hinein und erhalten die Distraktion aufrecht. Nach dem Entfernen des letzten Distraktors kann durch die zuletzt eingesetzte Führungshülse ein Eindrehinstrument eingeführt werden, an dessen distalem Ende das Implantat befestigt ist.

Das Eindrehen des Implantats ist insofern ein kritischer Schritt, als dadurch die axiale Position des Implantats im Bandscheibenfach endgültig festgelegt wird. Nimmt das Implantat nicht die gewünschte axiale Sollposition ein, so kann dies beim Patienten erneute Beschwerden verursachen und beispielsweise zu krankhaften Veränderungen in benachbarten Wirbelsäulensegmenten führen. Vor allem dann, wenn das Implantat zu weit in das Bandscheibenfach eingedreht wird, besteht überdies die Gefahr, dass Nervenstränge, Blutbahnen oder anderes empfindliches Gewebe durch das Implantat beschädigt werden.

Erschwerend kommt hinzu, dass der Chirurg das Implantat beim Eindrehen von außen im Allgemeinen nicht sehen kann. Eine kontinuierliche Kontrolle des gesamten Eindrehvorgangs mit Hilfe eines Fluoroskops oder anderer bildgebender Verfahren ist zwar möglich, aber aus Gründen der Strahlenbelastung problematisch. Deswegen wäre es optimal, wenn der Chirurg das Implantat möglichst präzise auch ohne Zuhilfenahme bildgebender Verfahren, d. h. lediglich unter Verwendung des ihm zur Verfügung stehenden Instrumentariums, eindrehen kann, bis es seine Sollposition erreicht hat. Eine Überwachung durch bildgebende Verfahren erfolgt dann vorzugsweise nur noch subsidiär, etwa um am Ende des Eindrehvorgangs eine Abschlusskontrolle durchzuführen.

Die bislang bekannten Eindrehinstrumente verfügen üblicherweise an ihrem distalen Ende über eine spezielle Aufnahme, an der das Implantat drehfest und axial gesichert befestigt werden kann. Nach Abschluss des Eindrehvorgangs wird diese Befestigung gelöst, so dass das Eindrehinstrument aus der Führungshülse herausgezogen werden kann. Einige Eindrehinstrumente sind dabei mit verstellbaren Anschlägen versehen, die an der Führungshülse anschlagen und ein weiteres Eindringen verhindern, wenn das Implantat eine vom Chirurgen festgelegte axiale Sollposition erreicht hat.

In der US 2006/0116688 A1 ist beispielsweise ein Anschlag beschrieben, der an einem Schaft eines Eindrehinstruments in verschiedenen Axialpositionen lösbar befestigt werden kann. Der Anschlag ist mit einem Sichtfenster versehen, durch das hindurch Zahlen erkennbar sind, die auf den Schaft aufgebracht sind. Die Zahlen zeigen dabei an, wie weit das distale Ende des Schafts aus der Führungshülse hervorragt, wenn der Anschlag an der Führungshülse anschlägt.

Nachteilig bei den bekannten Anschlägen ist allerdings, dass zwar ein zu tiefes Eindringen des Implantats in das Bandscheibenfach verhindert wird, der Chirurg aber während des Eindrehvorgangs nur sehr ungenau ermitteln kann, wie weit das Implantat noch von seiner Sollposition entfernt ist. Denn im Allgemeinen wird der Chirurg den Anschlag so einstellen, dass damit lediglich eine Gefährdung empfindlichen Gewebes verhindert wird. Die gewünschte Sollposition liegt im Allgemeinen jedoch etwas weniger weit vom Chirurgen entfernt, und das Erreichen dieser Sollposition kann der Chirurg nicht am Instrument ablesen. Zwar könnte der Chirurg natürlich den Anschlag auch so axial festlegen, dass in der Endposition des Eindrehinstruments das Implantat seine Sollposition erreicht. Dann wäre es dem Chirurgen jedoch nicht mehr möglich, kleinere Justierungen auch zum distalen Ende hin vorzunehmen, ohne den Anschlag zu lösen und auf diese Weise Gefahr zu laufen, versehentlich doch zu tief in das Bandscheibenfach einzudringen.

### ZUSAMMENFASSUNG DER ERFINDUNG

Aufgabe der Erfindung ist es deswegen, ein Instrumentarium zum Eindrehen eines mit einem Außengewinde versehenen Implantats in ein Bandscheibenfach anzugeben, mit dem das Implantat sicherer bis zum Erreichen der vorgegebenen Sollposition eingedreht werden kann.

Erfindungsgemäß wird diese Aufgabe durch ein Instrumentarium gelöst, das eine Führungshülse aufweist, die ein distales Ende hat und sich während des Eindrehvorgangs an Wirbeln abstützt, die das Bandscheibenfach begrenzen. Die Führungshülse hat ferner ein proximales Ende, das während des Eindrehvorgangs zum Chirurgen weist. Zum Instrumentarium gehört ferner ein Eindrehinstrument, das einen Schaft hat, der in die Führungshülse einführbar ist. Der Schaft hat ein distales Ende, an dem das Implantat drehfest festlegbar ist, und ein proximales Ende, an dem ein Drehgriff drehfest befestigt ist und das während des Eindrehvorgangs über das proximale Ende der Führungshülse hinausragt. Erfindungsgemäß weist das Eindrehinstrument eine Restvorschubanzeige auf, die dem Chirurgen während des Eindrehvorgangs quantitativ anzeigt, wie weit das Implantat in axialer Richtung von einer axialen Sollposition entfernt ist, die relativ zu dem Ort festgelegt ist, an dem sich die Führungshülse an den Wirbeln abstützt.

Die erfindungsgemäße Restvorschubanzeige ermöglicht es dem Chirurgen, sich der axialen Sollposition des Implantats vorsichtig anzunähern und das Implantat dort auch ohne Zuhilfenahme bildgebender Verfahren zu positionieren. Anstatt sich also nur auf einen Anschlag zu verlassen, der erst bei Erreichen einer kritischen axialen Tiefenposition wirksam wird, kann der Chirurg anhand der Restvorschubanzeige erkennen, wie weit er von der Sollposition entfernt ist, und den Eindrehvorgang genau dann abbrechen, wenn die Sollposition erreicht ist. Das bisherige vergleichsweise ungenaue Arbeiten in der Nähe einer Anschlagsposition wird erfindungsgemäß ersetzt durch ein gezieltes und vorsichtiges Anfahren der Sollposition, was nur durch die erfindungsgemäße Restvorschubanzeige ermöglicht wird.

Unter einer quantitativen Anzeige wird in diesem Zusammenhang insbesondere verstanden, dass der Chirurg durch Striche einer Skala oder durch sonstige Markierungen gleicher Teilung erkennen kann, wie viele Teilungseinheiten noch bis zum Erreichen der Sollposition zurückgelegt werden müssen. Selbstverständlich können die Markierungen auch durch Ziffern oder sonstige Symbole, etwa eine Folge absteigender Großbuchstaben (E, D, C, B, A), ergänzt oder ersetzt sein. Auch Striche oder Markierungen ungleicher Teilung können für die quantitative Anzeige des Restvorschubs verwendet werden, solange gewährleistet ist, dass der Chirurg den Restvorschub bis zum Erreichen der Sollposition zuverlässig abschätzen kann. So kann beispielsweise gerade eine zunehmend enger werdende Teilung dem Chirurgen deutlich machen, dass sich das Implantat immer mehr seiner Sollposition nähert.

Die Restvorschubanzeige kann derart ausgebildet sein, dass sie auch ein Überschreiten der Sollposition in axialer Richtung quantitativ anzeigt. Auf diese Weise erhält der Chirurg die Möglichkeit, eine Feinjustierung der axialen Lage des Implantats vorzunehmen und dabei stets an der Restvorschubanzeige erkennen zu können, wie weit sich das Implantat in beiden Richtungen von seiner Sollposition entfernt hat.

Die Restvorschubanzeige kann eine Skala umfassen, die auf den Schaft oder auf ein daran axial festgelegtes Bauteil aufgebracht ist und mit der Führungshülse, und zwar insbesondere mit deren proximalen Ende, zusammenwirkt. Die Führungshülse kann zu diesem Zweck beispielsweise mit einem Fenster versehen sein, durch das hindurch die Skala auf dem Schaft erkennbar ist und an dessen Rand eine Markierung angebracht ist, die auf die Skala weist und dadurch den Restvorschub anzeigt. Die Skala kann hierzu z. B. mit Zahlen beschriftet sein, welche in einer vorgegebenen Längeneinheit den axialen Abstand zwischen dem Implantat und der Sollposition angeben.

Am einfachsten ist es jedoch, wenn das proximale Ende der Führungshülse direkt oder eine dort angebrachte (z. B. zeigerartige) Markierung auf die Skala weist und dadurch den Restvorschub anzeigt, wenn das Eindrehinstrument in die Führungshülse eingeführt ist.

Das Eindrehinstrument kann eine relativ zu dem Schaft axial beweglich geführte Anzeigehülse aufweisen, die während des Einführens des Eindrehinstruments in die Führungshülse oder zumindest im Verlauf des Eindrehvorgangs an dem proximalen Ende der Führungshülse anschlägt und mit einem Sichtfenster versehen ist, durch das hindurch die Skala erkennbar ist. Auf diese Weise wird die Skala gewissermaßen in axialer Richtung zum proximalen Ende, d. h. zum Chirurgen hin, verlegt. Dies ist insofern vorteilhaft, als dadurch der Chirurg die Skala im Allgemeinen leichter ablesen kann, als wenn sie sich im Bereich der Führungshülse befindet.

Der Rand des Sichtfensters kann hierzu mit einer z. B. zeigerartigen Markierung versehen sein, die auf die Skala gerichtet ist.

Ferner kann das Eindrehinstrument ein elastisches Element aufweisen, das die Anzeigehülse in axialer Richtung derart vorspannt, dass die Anzeigehülse während des Eindrehvorgangs gegen die Wirkung des elastisches Elements axial relativ zu dem Schaft verlagert wird. Die Vorspannung gewährleistet somit, dass die Anzeigehülse nach dem erstmaligen Anschlagen an der Führungshülse in dieser Position verbleibt und sich nicht unbeabsichtigt wieder von der Führungshülse entfernt. Die Skala kann einen ersten Skalenabschnitt, der mit der Markierung auf der Führungshülse zusammenwirkt, und einen zweiten Skalenabschnitt aufweisen, der mit der Markierung auf der Anzeigenhülse zusammenwirkt.

Insbesondere können die beiden Skalenabschnitte derart aufeinander abgestimmt sein, dass dann, wenn die Anzeigehülse während des Eindrehvorgangs an dem proximalen Ende der Führungshülse anschlägt, nur noch der zweite Skalenabschnitt den Restvorschub und gegebenenfalls ein Überschreiten der Sollposition in axialer Richtung quantitativ anzeigt. Durch einen solchen Wechsel der Restvorschubanzeige zwischen den unterschiedlichen Skalenabschnitten wird der Chirurg zusätzlich darauf aufmerksam gemacht, dass sich das Implantat nun in unmittelbarer Nähe der Sollposition befindet und der weitere Eindrehvorgang mit besonderer Vorsicht durchgeführt werden sollte.

Der Restvorschub kann weniger als -5 mm, vorzugsweise weniger als -2.5 mm, betragen, wenn während des Eindrehvorgangs die Anzeigehülse an dem proximalen Ende der Führungshülse anschlägt und dadurch der Restvorschub auf dem zweiten Skalenabschnitt erkennbar ist.

Die Führungshülse kann an einem distalen Ende zwei Distanzzungen haben, die in das Bandscheibenfach eingreifen, wenn sich die Führungshülse während des Eindrehvorgangs an den Wirbeln abstützt.

Der Schaft des Eindrehinstruments kann eine sich über die gesamte Länge des Schafts erstreckende Durchgangsbohrung haben. Ferner kann das Eindrehinstrument eine Befestigungsstange aufweisen, die in der Durchgangsbohrung geführt und an ihrem distalen Ende mit einem Befestigungsabschnitt versehen ist, mit dem das Implantat in axialer Richtung an der Befestigungsstange fixierbar ist.

Der Befestigungsabschnitt kann hierzu ein Außengewinde aufweisen, das dazu eingerichtet ist, mit einem komplementären Innengewinde zusammenzuwirken, das in einer Bohrung am Implantat ausgebildet ist.

Ferner kann die Befestigungsstange an ihrem proximalen Ende einen Feststellgriff tragen, bei dessen Betätigung sich die Befestigungsstange um ihre Längsachse dreht.

Der dem Schaft zugeordnete Drehgriff kann lösbar an dem Schaft befestigt sein. Dadurch ist es möglich, ein und denselben Drehgriff an unterschiedlich dimensionierten Schäften zu befestigen.

Das Eindrehinstrument kann zum Befestigen des Drehgriffs am Schaft einen Rastmechanismus aufweisen, wobei die Verrastung des Drehgriffs mit dem Schaft nur durch Betätigen einer federbeaufschlagten Rasttaste lösbar ist. Auf diese Weise wird verhindert, dass sich der Drehgriff unbeabsichtigt vom Schaft löst und wieder neu montiert werden müsste.

Die drehfeste Festlegung des Drehgriffs am Schaft kann durch Formschluss erzielt werden, so dass der Rastmechanismus den Drehgriff nur noch in axialer Richtung relativ zum Schaft fixieren muss. Zur Erzielung des Formschlusses können an dem Drehgriff und an dem Schaft Planflächen vorgesehen sein, die derart ausgerichtet sind, dass sich der Drehgriff seitlich auf den Schaft aufschieben lässt und dieser im aufgeschobenen Zustand drehfest und formschlüssig am Schaft festgelegt ist.

Der Rastmechanismus kann dann beispielsweise eine auf den Schaft aufgeschraubte Rasthülse aufweisen, die im verrasteten Zustand axial fixiert und gegebenenfalls auch drehfest an dem Schaft festgelegt ist.

Weiter kann der Rastmechanismus so eingerichtet sein, dass eine Verrastung erst eintritt, wenn der Rastmechanismus eine vorgegebene Zahl von deutlich hörbaren (Schalldruck insbesondere > 20 dB) Klickgeräuschen erzeugt hat. Die Klickgeräusche zeigen dann dem Chirurgen oder einem Assistenten an, dass die Verrastung eingetreten ist und der Drehgriff sicher am Schaft befestigt wurde.

Gegenstand der Erfindung ist ferner ein Implantationssystem mit einem mit einem Außengewinde versehenen Implantat, das zum Eindrehen in ein Bandscheibenfach eingerichtet ist, und mit einem erfindungsgemäßen Instrumentarium. Das Implantat hat in zwei orthogonalen Richtungen, die senkrecht zu einer Längsachse des Implantats verlaufen, unterschiedliche Abmessungen, und zwar derart, dass sich je nach Winkelorientierung des Implantats im Bandscheibenfach unterschiedliche Abstände und/oder Winkel zwischen den das Bandscheibenfach begrenzenden Wirbeln ergeben. Ein Befestigungsabschnitt des Eindrehinstruments ist derart eingerichtet, dass sich das Implantat nur in genau zwei Winkelorientierungen, die sich um 180° voneinander unterscheiden, an dem Eindrehinstrument drehfest festlegen lässt. Durch diese eindeutige Festlegung des Implantats am Eindrehinstrument kann die Orientierung des Implantats im Bandscheibenfach am Eindrehinstrument selbst erkannt werden, ohne dass bildgebende Verfahren erforderlich sind.

Zu diesem Zweck kann beispielsweise der Drehgriff am Eindrehinstrument einen Abschnitt aufweisen, der in einem Schnitt senkrecht zur Längsachse des Schafts zumindest im Wesentlichen die Form eines Rechtecks hat, wobei die langen Seiten des Rechtecks parallel zu der Richtung verlaufen, entlang der die Abmessungen des an dem Eindrehinstrument festgelegten Implantats größer sind.

Die drehfeste Festlegung des Implantats an dem Eindrehinstrument kann insbesondere durch Formschluss erzielt werden. Ferner kann ein Außengewinde am Befestigungsabschnitt derart axial angeordnet sein, dass es nur in den zwei Winkelorientierungen in ein Innengewinde am Implantat eingreifen kann. Das Implantationssystem kann außerdem mehrere Schäfte, die sich durch die Ausbildung der Befestigungsabschnitte voneinander unterscheiden, und mehrere Implantate unterschiedlicher Größe umfassen, die Befestigungsmittel haben, mit denen sie jeweils drehfest an dem Befestigungsabschnitt eines der Schäfte festlegbar sind. Mindestens zwei unterschiedlich große Implantate haben dabei auch unterschiedlich ausgebildete Befestigungsmittel. Ferner umfasst das System mehrere Führungshülsen, deren Innendurchmesser an die Durchmesser der Implantate und der Schäfte angepasst sind.

Das System kann auch mehrere Befestigungsstäbe umfassen, die an die unterschiedlichen Implantate angepasst sind. Vorzugsweise lassen sich dann unterschiedliche Eindrehinstrumente erhalten, indem einer von mehreren unterschiedlichen Befestigungsstäben, ein dazu passender Schaft und ansonsten identische Bauteile zusammengebaut werden.

### KURZE BESCHREIBUNG DER ZEICHNUNGEN

Weitere Merkmale und Vorteile der Erfindung ergeben sich aus der nachfolgenden Beschreibung eines Ausführungsbeispiels anhand der Zeichnungen. Darin zeigen:
- Figur 1: eine perspektivische Ansicht einer Führungshülse, die Teil des erfindungsgemäßen Instrumentariums ist;
- Figur 2: eine Vorderansicht der in der Figur 1 gezeigten Führungshülse;
- Figur 3: eine Seitenansicht der in der Figur 1 gezeigten Führungshülse;
- Figur 4: eine Untersicht der in der Figur 1 gezeigten Führungshülse;
- Figur 5: eine perspektivische Ansicht eines Eindrehinstruments, das ebenfalls Teil des erfindungsgemäßen Instrumentariums ist, zusammen mit einem einzudrehenden Implantat;
- Figur 6: eine perspektivische Explosionsansicht einer Baugruppe des in der Figur 5 gezeigten Eindrehinstruments;
- Figur 7: eine perspektivische Ansicht eines Teils der in der Figur 6 gezeigten Baugruppe im montierten Zustand;
- Figur 8: eine perspektivische Explosionsansicht des in der Figur 5 gezeigten Eindrehinstruments, jedoch ohne Griffeinheit;
- Figur 9: eine perspektivische Ansicht des in der Figur 5 gezeigten Eindrehinstruments, jedoch mit seitlich abgezogener Griffeinheit;
- Figur 10: einen axialen Schnitt durch das in der Figur 5 gezeigten Eindrehinstrument mit daran befestigtem Implantat;
- Figur 11: einen axialen Schnitt durch die Griffeinheit und die daran angrenzenden Teile des Eindrehinstruments vor Verrastung einer Rasthülse;
- Figur 12: einen Ausschnitt aus der Figur 11, jedoch nach Verrastung der Rasthülse;
- Figur 13: einen Querschnitt durch das in der Figur 5 gezeigte Eindrehinstrument auf der Höhe der Rasthülse;
- Figur 14: einen an die Figur 12 angelehnten Ausschnitt für eine alternative Ausgestaltung eines Rastmechanismus;
- Figur 15: eine Seitenansicht der in der Figur 1 gezeigten Führungshülse, jedoch eingeführt in ein Bandscheibenfach;
- Figur 16: eine Vorderansicht der in das Bandscheibenfach eingeführten Führungshülse gemäß der Figur 15;
- Figur 17: eine perspektivische Ansicht des in der Figur 5 gezeigten Eindrehinstruments und mehrerer unterschiedlicher Schäfte und Befestigungsstangen;
- Figur 18: eine perspektivische Ansicht des distalen Endes des Eindrehinstruments mit daran befestigtem Implantat;
- Figur 19: einen axialen Schnitt durch das in der Figur 18 gezeigte distale Ende;
- Figur 20: eine Vorderansicht des in die Führungshülse eingeführten Eindrehinstruments vor Beginn des Eindrehvorgangs;
- Figur 21: eine Vorderansicht des in das Bandscheibenfach eingedrehten Implantats in seiner axialen Sollposition mit aufgeschnittener Führungshülse;
- Figuren 22a bis 22e: Ausschnitte aus der Figur 20, jedoch in unterschiedlichen Axialpositionen des Eindrehinstruments.

### BESCHREIBUNG BEVORZUGTER AUSFÜHRUNGSBEISPIELE

### I. Aufbau

Das erfindungsgemäße Instrumentarium zum Eindrehen eines mit einem Außengewinde versehenen Implantats in ein Bandscheibenfach umfasst mehrere Führungshülsen und ein Eindrehinstrument, das durch den Austausch eines Schafts und einer Befestigungsstange an das jeweils ausgewählte Implantat angepasst wird. Im Folgenden wird zunächst der Aufbau einer der Führungshülsen beschrieben; die anderen Führungshülsen unterscheiden sich davon nur durch bestimmte Abmessungen.

### a) Führungshülse

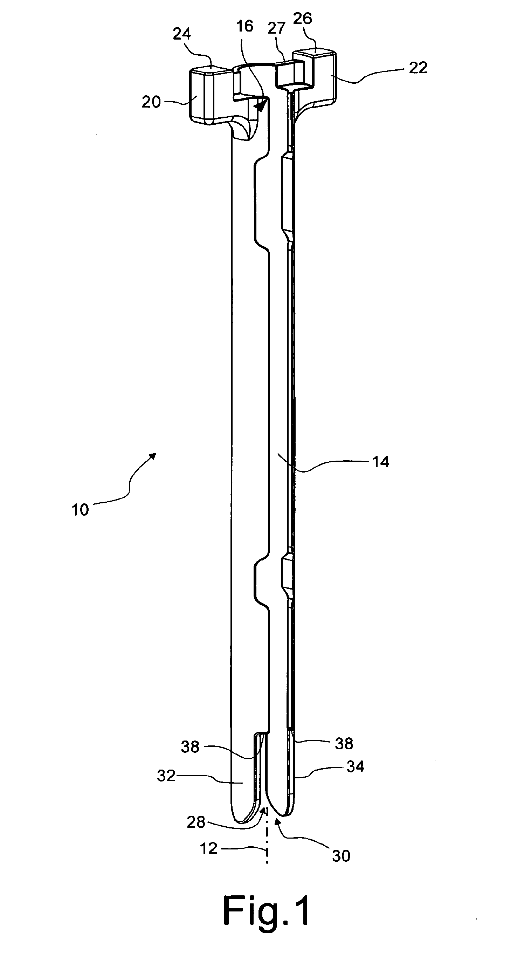

Der Aufbau einer der Führungshülsen wird im Folgenden mit Bezug auf die Figuren 1 bis 4 näher beschrieben. Diese Figuren zeigen die Führungshülse in einer perspektivischen Ansicht, einer Vorderansicht, einer Seitenansicht bzw. einer Untersicht.

Die insgesamt mit 10 bezeichnete Führungshülse hat ein proximales Ende, das in den Figuren nach oben weist und bei der Verwendung der Führungshülse 10 während einer Bandscheibenoperation dem Chirurgen zugewandt ist. Das gegenüberliegende, in den Figuren 1 bis 3 nach unten weisende Ende der Führungshülse 10 wird als distales Ende bezeichnet. Mit dem distalen Ende voran wird die Führungshülse 10 während der Operation durch eine vorab freigelegte Zugangsöffnung in den Patienten eingeführt.

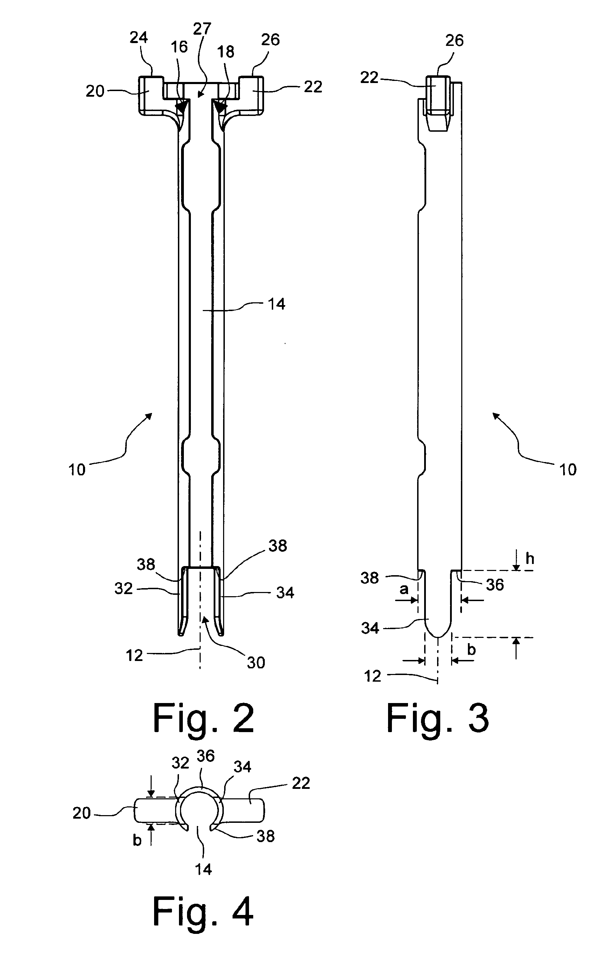

Die Führungshülse 10 hat im Wesentlichen einen kreisringförmigen Querschnitt; möglich sind jedoch auch andere, z. B. quadratische, Querschnitte. Parallel zu einer Längsachse 12 der Führungshülse 10 erstreckt sich ein Längsschlitz 14, und zwar durchgehend vom proximalen Ende bis zum distalen Ende der Führungshülse 10. Wie am besten in der Figur 2 erkennbar ist, sind am proximalen Ende der Führungshülse 10 zwei Markierungen 16, 18 angeordnet, welche die Form von Pfeilspitzen haben und auf die Führungshülse 10 aufgedruckt oder darin eingraviert sein können. Im Bereich der Markierungen 18, 20 sind zwei Vorsprünge 20, 22 ausgebildet, die sich diametral einander gegenüberliegen und radial von der Längsachse 12 der Führungshülse 10 abstehen. Die proximalen Endflächen der beiden Vorsprünge 20, 22 bilden Einwirkflächen 24, 26, die eben sind und senkrecht zur Längsachse 12 der Führungshülse 10 verlaufen. Die Einwirkflächen 24, 26 können dazu verwendet werden, mit einem Hammer oder einem anderen Einschlaginstrument das distale Ende der Führungshülse 10 in das Bandscheibenfach zu treiben. Dies gelingt auch dann, wenn von dem proximalen Ende der Führungshülse 10 ein Instrument, z.B. ein Distraktor oder ein Eindrehinstrument, hervorsteht, das zuvor in die Führungshülse 10 eingeführt wurde. Zwischen den Vorsprünge 20, 22 erstreckt sich ein Steg 27, der in axialer Richtung etwas niedriger ist als die Vorsprünge 20, 22.

An ihrem distalen Ende weist die Führungshülse 10 zwei diametral einander gegenüberliegende Aussparungen 28, 30 auf, zwischen denen zwei Distanzzungen 32, 34 verbleiben, die einander gegenüberliegen. Die Distanzzungen 32, 34 werden somit durch einen Abschnitt der Führungshülsenwand gebildet, sind aber zum distalen Ende hin annähernd elliptisch abgerundet, wie man dies am besten in der Seitenansicht der Figur 3 erkennen kann.

Die proximale Stirnfläche der Aussparung 28 bildet eine erste Auflageschulter 36 (siehe Figuren 3 und 4), während die gegenüberliegende Aussparung 30 infolge des dort verlaufenden Längsschlitzes 14 zwei schmalere zweite Auflageschultern 38 festlegt.

Für die Erläuterung der für die Funktion wichtigen Abmessungen der Distanzzungen 32, 34 wird im Folgenden Bezug auf die Figuren 3 und 4 genommen. Entlang einer Transversalrichtung, die senkrecht zur Längsachse 12 verläuft, haben die beiden Distanzzungen 32, 34 eine Breite b und entlang der Längsrichtung 12 eine Höhe h. Die Führungshülsen des erfindungsgemäßen Systems unterscheiden sich voneinander durch die Breiten b der Distanzzungen 32, 34, aber auch durch den Innen- und Außendurchmesser der Führungshülsen 10.

### b) Eindrehinstrument

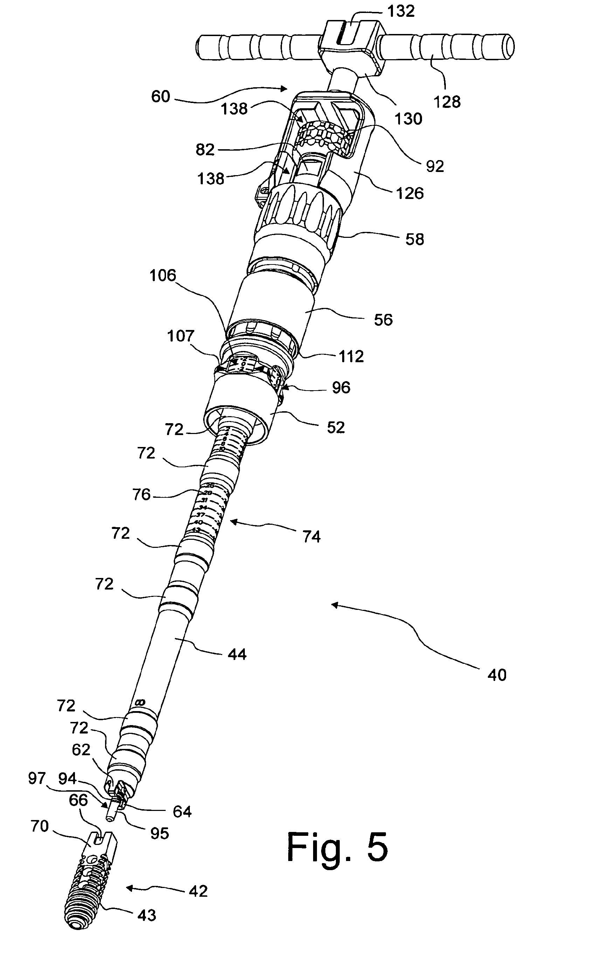

Im Folgenden wird der Aufbau eines erfindungsgemäßen Eindrehinstruments mit Bezug auf die Figuren 5 bis 13 erläutert, die das insgesamt mit 40 bezeichnete Eindrehinstrument in verschiedenen Ansichten und Schnitten zeigen. Die Beschreibung beginnt mit einem Überblick über die wesentlichen Bauteile des Eindrehinstruments 40. Daran schließt sich eine detaillierte Darstellung des Aufbaus und des Zusammenwirkens dieser Bauteile an.

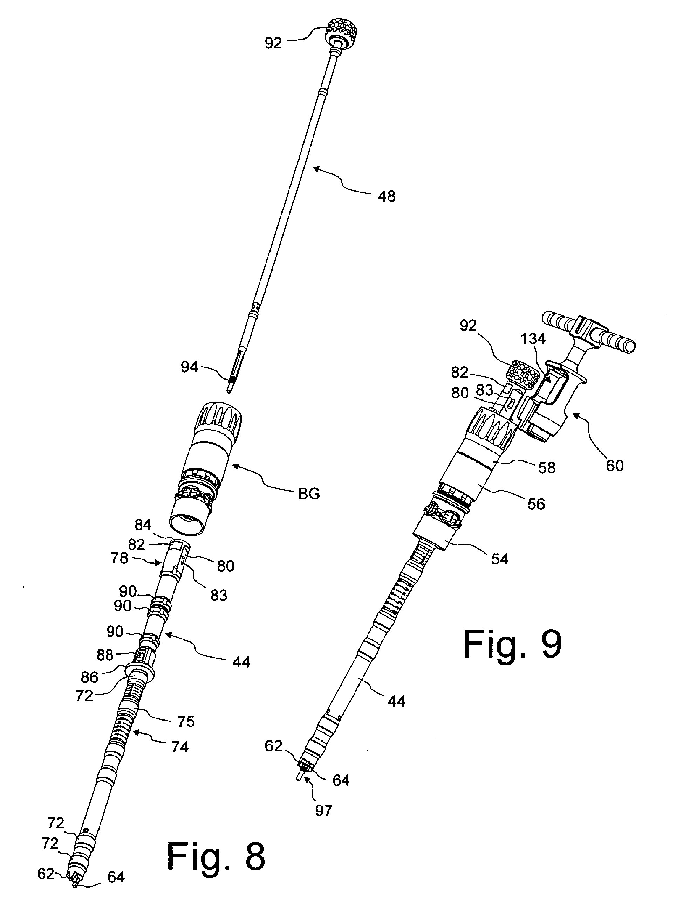

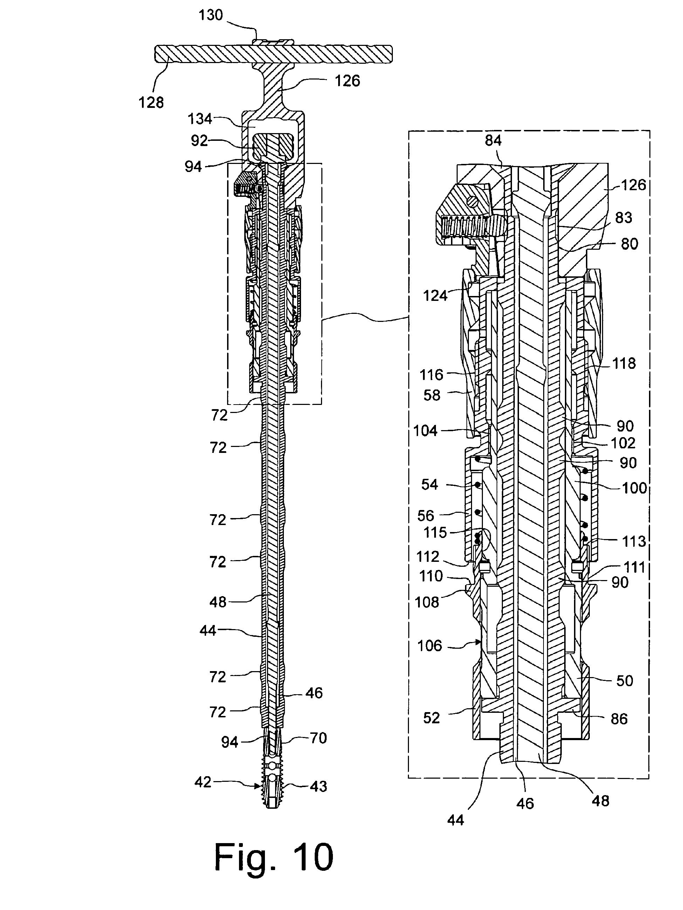

Zunächst wird auf die Figur 5 Bezug genommen, die in einer perspektivischen Darstellung das fertig montierte Eindrehinstrument 40 sowie ein einzudrehendes Implantat 42 mit Außengewinde 43 zeigt. Das Eindrehinstrument 40 weist einen Schaft 44 auf, der mit einer axialen Durchgangsbohrung 46 versehen ist, wie dies am besten in dem axialen Schnitt der Figur 10 erkennbar ist. In der Durchgangsbohrung 46 des Schafts 44 ist eine Befestigungsstange 48 geführt, die isoliert gut in der Explosionsdarstellung der Figur 8 erkennbar ist.

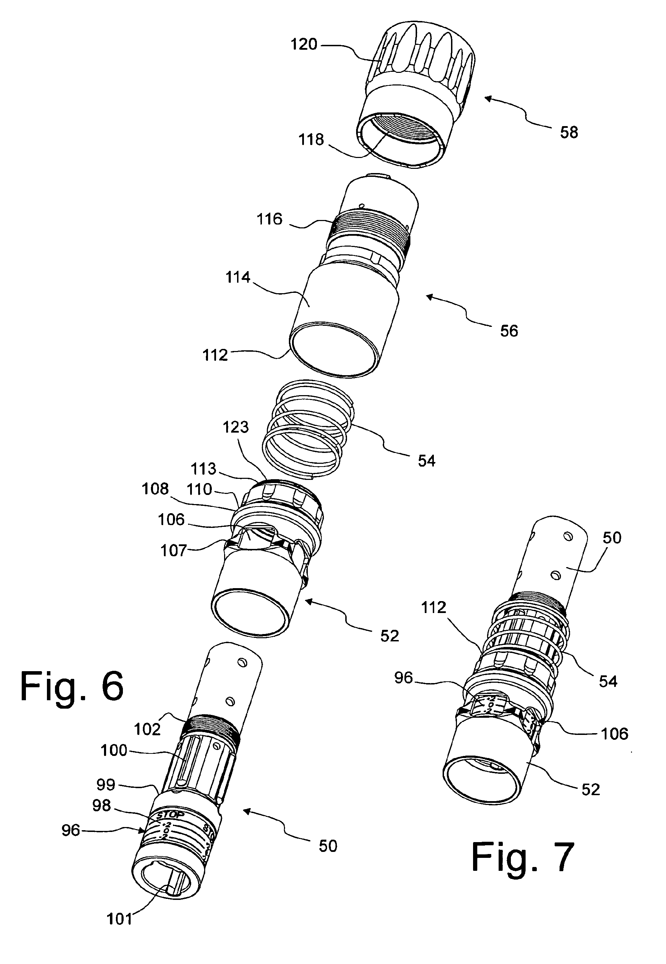

Auf den Schaft 44 ist von seinem proximalen, in der Figur 8 nach oben weisenden Ende her eine Innenhülse 50 aufgeschoben, die isoliert am besten unten in der Figur 6 erkennbar ist, auf die auch im folgenden weiter Bezug genommen wird. Nach dem Aufschieben ist die Innenhülse 50 drehfest am Schaft 44 festgelegt. Auf der Innenhülse 50 ist axialbeweglich eine Anzeigehülse 52 geführt, die sich unter dem Druck einer Druckfeder 54 an der Innenhülse 50 abstützt. Die Druckfeder 54 ist wiederum an einer Anschlagshülse 56 festgelegt, die mit der Innenhülse 50 verschraubt ist. Auf die Anschlagshülse 56 ist eine Rasthülse 58 aufgeschraubt, mit der die Innenhülse 50, die Anzeigehülse 52, die Druckfeder 54 und die Anschlagshülse 56 gegenüber einer etwa in den Figur 5 und 9 erkennbaren Griffeinheit 60 verspannt werden, die gegen axiale Verschiebung gesichert seitlich auf den Schaft 44 aufgeschoben ist.

Im Folgenden werden die vorstehend beschriebenen Komponenten des Eindrehinstruments 40 und deren Zusammenwirken näher beschrieben.

Der Schaft 44, der vollständig am besten in der Explosionsdarstellung der Figur 8 erkennbar ist, weist an seinem distalen, in der Figur 8 nach unten zeigenden Ende zwei Führungsnasen 62, 64 auf. Wie man am besten in der Figur 5 erkennen kann, haben die Führungsnasen 62, 64 eine Form, die komplementär zur Geometrie von Führungsnuten 66 sind, die in einem Basisabschnitt 70 des Implantats 42 ausgebildet sind. Dieser hat einen rechteckigen Querschnitt, d. h. die Abmessungen in orthogonalen und zur Längsachse senkrechten Richtungen sind unterschiedlich.

Ferner sind am Schaft 44 mehrere Führungsringe 72 ausgebildet, in deren Bereichen der Durchmesser des Schafts 44 vergrößert ist. Der Durchmesser der Führungsringe 72 ist dabei geringfügig kleiner als der Innendurchmesser der Führungshülse 10, so dass der Schaft 44 mit kleinem Spiel in die Führungshülse eingeführt werden kann und dort axial beweglich bleibt.

Etwa in der Mitte des Schafts 44 befindet sich ein erster Skalenabschnitt 74, der in einem Bereich 75 durch einen der Führungsringe 72 unterbrochen ist. In der Figur 5 ist erkennbar, dass die dort auf den Schaft 44 aufgebrachte Skala mit Zahlen 76 versehen ist, deren Bedeutung später im Zusammenhang mit der Funktionsbeschreibung erläutert wird.

Am proximalen Ende des Schafts 44 befindet sich ein Kopfabschnitt 78, dessen Form am besten in der Figur 8 erkennbar ist. Der Kopfabschnitt 78 ist mit einem ersten Parallelflach 80 mit größeren Planflächen und um 90° verdreht hierzu mit einem zweiten Parallelflach 82 mit kleineren Planflächen versehen. An den größeren Planflächen des ersten Parallelflachs 80 sind dabei kurze, sich in axialer Richtung erstreckende Nuten 83 eingearbeitet. Am distalen Ende trägt der Kopfabschnitt 78 einen Abschlussring 84, dessen Durchmesser kleiner als der Innendurchmesser der Innenhülse 50 ist, so dass diese über das proximale Ende des Schafts 44 aufgeschoben werden kann.

In der Figur 8 ist zwischen dem ersten Skalenabschnitt 74 und dem Kopfabschnitt 78 ein am Schaft 44 ausgebildeter Stützring 86 erkennbar, dessen Durchmesser größer als der Innendurchmesser der Innenhülse 50 ist. Axial unmittelbar benachbart sind am Schaft 44 kurze, sich in axialer Richtung erstreckende Führungsrippen 88 ausgebildet, die in noch näher zu erläutender Weise mit der Innenhülse 50 zusammenwirken. Zwischen den Führungsrippen 88 und dem Kopfabschnitt 78 sind zweite Führungsringe 90 angeordnet, deren Durchmesser etwas kleiner als der Innendurchmesser der Innenhülse 50 ist. Dadurch kann die Innenhülse 50 axial geführt und mit ausreichendem Spiel auf den Schaft 44 aufgeschoben werden. Gegenüber einem insgesamt dicker ausgebildeten Schaft 44 wird auf diese Weise eine Materialersparnis und eine Gewichtsverringerung erzielt.

Die am besten in der Figur 8 erkennbare Befestigungsstange 48, die sich im montierten Zustand durch die Durchgangsbohrung 46 des Schafts 44 erstreckt, ist an ihrem proximalen Ende mit einem gerändelten Feststellgriff 92 und in der Nähe des distalen Endes mit einem Außengewinde 94 versehen. Das Außengewinde 94 ist dabei etwas beabstandet vom distalen Ende der Befestigungsstange 48 angeordnet, wie dies gut in der Figur 5 erkennbar ist. Im montierten Zustand ragen der mit dem Außengewinde 94 versehene Abschnitt und der sich distal daran anschließende gewindefrei Abschnitt 95 aus dem Schaft 44 hervor und bilden gemeinsam einen Stummel 97.

Im Folgenden wird, sofern nicht anders angegeben, Bezug auf die Figur 6 genommen. Die Innenhülse 50 ist in der Nähe ihres distalen Endes mit einem zweiten Skalenabschnitt 96 versehen und mit Zahlen 98 beschriftet. Zum proximalen Ende hin endet der zweiten Skalenabschnitts 96 mit der Beschriftung STOP.

Oberhalb des zweiten Skalenabschnitts 96 weist die Innenhülse 50 einen umlaufenden Absatz 99 auf, an den sich umfangsseitig vier in axialer Richtung erstreckende Führungsrippen 100 anschließen. Darauf folgt ein Außengewinde 102, das mit einem Innengewinde 104 zusammenwirkt, das an der Anschlagshülse 56 innenseitig ausgebildet und nur im axialen Schnitt der Figur 10 erkennbar ist.

Innenseitig ist die Innenhülse 50 mit zwei diametral einander gegenüberliegenden Längsnuten 101 versehen.

Die Anzeigehülse 52 hat vier Sichtfenster 106 mit annähernd rechteckiger Kontur. Zwischen den Sichtfenstern sind von außen Markierungen 107 in der Art von Pfeilspitzen auf die Anzeigehülse 52 aufgebracht. Ferner weist die Anzeigehülse 52 einen umlaufenden Kragen 108 auf, der an seiner proximalen Seite eine Schulter 110 bildet, auf die eine in der Figur 10 erkennbare flache Ringnut 111 folgt. Die Schulter 110 schlägt an einer ringförmigen Stirnfläche 112 der Anschlagshülse 56 an, wenn die Anzeigehülse 52 axial ausreichend weit gegen den Widerstand der Druckfeder 54 verschoben wird. Die Druckfeder 54 stützt sich dabei an einem Absatz 113 ab, der am proximalen Ende der Anzeigehülse 52 ausgebildet ist.

Innenseitig ist die Anzeigehülse 52 an ihrem proximalen Ende mit vier äquidistanten, in der Figur 10 bei 115 angedeuteten Nuten versehen, die sich axial erstrecken und komplementär zu den Führungsrippen 100 auf der Innenhülse 50 ausgebildet sind. Wird die Anzeigehülse 52 auf die Innenhülse 50 aufgeschoben, so stellen die Nuten 115 und die Führungsrippen 100 sicher, dass die Anzeigehülse 52 drehfest, aber axial beweglich an der Innenhülse 50 festgelegt ist.

Die Anschlagshülse 56 hat einen distalen Abschnitt 114, dessen Innendurchmesser so bemessen ist, dass er über das proximale Ende der Innenhülse 50, den proximal jenseits des Kragens 108 verbleibenden Abschnitt der Anzeigehülse 52 und über die Druckfeder 54 geschoben werden kann. Die Anschlagshülse 56 trägt ferner ein Außengewinde 116, das mit einem an der Rasthülse 58 ausgebildeten Innengewinde 118 verschraubbar ist.

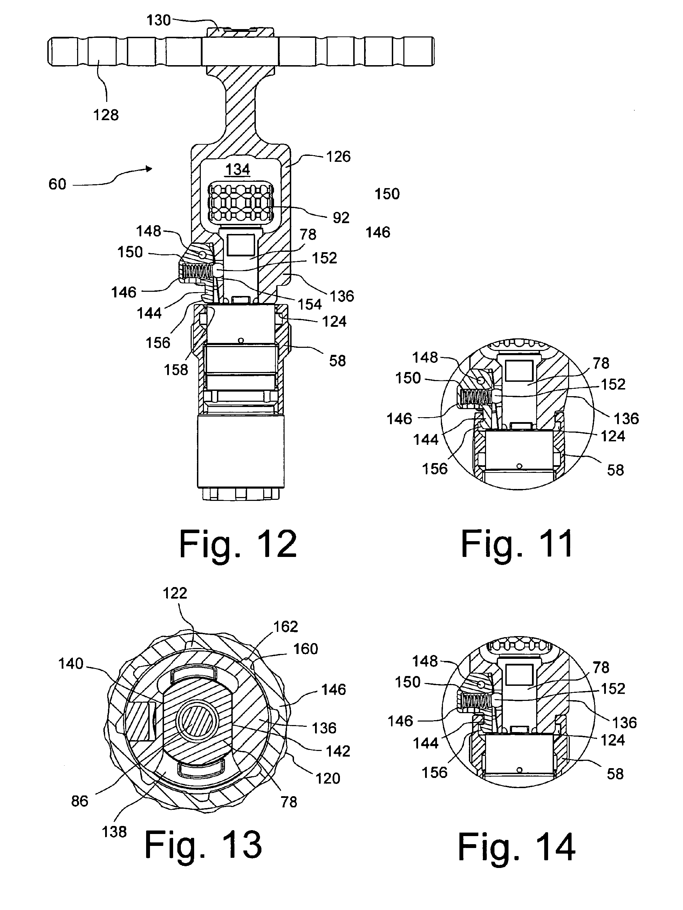

Außenseitig ist die Rasthülse 58 mit einer Rändelung 120 versehen, um sie beim Aufschrauben auf die Anschlagshülse 56 sicher fassen zu können. An ihrem proximalen Ende ist die Rasthülse 58 innenseitig mit mehreren äquidistanten Nuten 122 versehen, die am besten in dem Querschnitt der Figur 13 erkennbar sind. Unterhalb der Nuten 122 weist die Wandung der Rasthülse 58 eine in der Figur 10 erkennbare umlaufende Hinterschneidung 124 auf, die zusammen mit den Nuten 122 in noch näher darzustellender Weise eine Verrastung mit der Griffeinheit 60 ermöglicht.

Die am besten in dem axialen Schnitt der Figur 11 erkennbare Griffeinheit 60 umfasst ein Griffgehäuse 126, an dem einstückig ein Drehgriff 128 ausgebildet ist. Der bei 130 angedeutete Verbindungsabschnitt zwischen dem Drehgriff 128 und dem Griffgehäuse 126 ist im Querschnitt rechteckig, wie dies am besten in der Figur 5 erkennbar ist. Außenseitig sind auf dem Verbindungsabschnitt 130 zwei streifenartige Nuten 132 ausgebildet, die, wie ebenfalls in der Figur 5 erkennbar ist, im montierten Zustand des Eindrehinstruments 40 genauso ausgerichtet sind wie die Führungsnasen 62, 64 am distalen Ende des Schafts 44.

Das Griffgehäuse 126 der Griffeinheit 60 hat, wie man am besten in den Figuren 9 und 11 erkennen kann, ein großes Fenster 134, das der Aufnahme des an der Befestigungsstange 48 ausgebildeten Feststellgriffs 92 dient, so dass dieser auch im montierten Zustand betätigbar bleibt. An das Fenster 134 schließt sich eine einseitig offene Manschette 136 an, deren etwa in den Figuren 5 und 13 erkennbare Öffnung 138 derart bemessen ist, dass die Griffeinheit 60 seitlich auf den Kopfabschnitt 78 des Schafts 44 aufgesetzt werden kann (vgl. Figur). Wie der Querschnitt der Figur 13 zeigt, liegen planparallele Innenflächen 140, 142 der Manschette 136 dabei formschlüssig an den Planflächen des ersten Parallelflachs 80 des Kopfabschnitts 78 an, wodurch die Griffeinheit 60 drehfest am Schaft 44 festgelegt ist.

An der Manschette 136 des Griffgehäuses 126 ist außerdem ein mit einem Rasthaken 144 versehener Schwenkhebel 146 angebracht, der, zusammen mit den Nuten 122 und den Hinterschneidungen 124 der Rasthülse 58, einen Rastmechanismus bildet, damit die Griffeinheit 60 gegen ein unbeabsichtigtes seitliches Abziehen gesichert ist. Der um eine Schwenkachse 148 verschwenkbare Schwenkhebel 146 wird dabei von einer kleinen Druckfeder 150 vorgespannt, die sich an einer Kugel 152 abstützt. Die Kugel 152 ragt ihrerseits aus einer Öffnung 154 in der Manschette 136 infolge des von der Druckfeder 150 ausgeübten Druckes ein wenig heraus, weicht aber auf gegen die Druckfeder 150 wirkenden Druck in die Öffnung 154 zurück. Wenn die Griffeinheit 60 wie in der Figur 11 gezeigt auf den Kopfabschnitt 78 des Schafts 44 aufgesetzt ist, greift die Kugel 152 in eine der Nuten 83 ein, die am ersten Parallelflach 80 des Kopfabschnitts 78 ausgebildet sind. Die mit den Nuten 83 zusammenwirkende druckbeaufschlagte Kugel 154 bildet somit eine Verliersicherung, die gewährleistet, dass die seitlich auf den Kopfabschnitt 78 aufgeschobene Griffeinheit 60 sich beim Zusammenbau des Eindrehinstruments 40 nicht sofort wieder unbeabsichtigt vom Kopfabschnitt 78 löst, sobald die Griffeinheit 60 losgelassen wird.

### II. Zusammenbau des Eindrehinstruments

Im Folgenden wird der Zusammenbau des Eindrehinstruments 40 erläutert.

In einem ersten Schritt wird die Anzeigehülse 52 auf die Innenhülse 50 aufgeschoben, wobei die Führungsrippen 100 an der Innenhülse 50 in die an der Innenseite der Anzeigehülse 52 ausgebildeten korrespondierenden Nuten eingreifen. Um eine der vier möglichen winkelmäßigen Relativpositionen zu finden, muss dabei die Anzeigehülse 52 eventuell ein wenig verdreht werden. Ein in den Figuren nicht erkennbarer ringförmiger Absatz an der Innenfläche der Anzeigehülse 52 schlägt dabei an dem Absatz 99 an, der an der Innenhülse 50 ausgebildet ist. Anschließend wird die Druckfeder 54 über das proximale Ende der Innenhülse 50 geführt, bis sie am Absatz 113 der Anzeigehülse 52 aufsitzt und leicht einrastet. Dieser Montagezustand ist in der Figur 7 gezeigt.

In einem nächsten Schritt wird die Anschlagshülse 56 mit dem distalen Abschnitt 114 voran auf die in der Figur 7 gezeigte Baugruppe aufgesetzt und mit dieser verschraubt, wobei das Innengewinde 104 der Anschlagshülse 56 mit dem Außengewinde 102 der Innenhülse 50 in Eingriff kommt und die Druckfeder 54 innen an der Anschlagshülse 56 anschlägt. Die Innenhülse 50 und die Anschlagshülse 56 sind nun fest miteinander verbunden, während die Anzeigehülse 52 axial durch die Führung an den Führungsrippen 100 beweglich bleibt. Die Anzeigehülse 52 kann somit gegen den Widerstand der Druckfeder 54 zur distalen Stirnfläche 112 der Anschlagshülse 56 verschoben werden. Bei einer solchen axialen Verschiebung streichen die an der Anzeigehülse 52 ausgebildeten Sichtfenster 106 über den zweiten Skalenabschnitt 96, der auf die Innenhülse 50 aufgebracht ist. Die zwischen den Sichtfenstern 106 angebrachten Markierungen 107 auf der Anzeigehülse 52 zeigen dabei auf die Skala und die Zahlen 98.

In einem weiteren Schritt wird die Rasthülse 58 auf die Anschlagshülse 56 aufgeschraubt, wobei das Innengewinde 118 der Rasthülse 58 mit dem Außengewinde 116 der Anschlagshülse 56 in Eingriff kommt. Die dadurch erhaltene Baugruppe ist in der Mitte der Figur 8 unterhalb der Befestigungstange 48 dargestellt.

Die Baugruppe BG wird nun über das proximale Ende des Schafts 44 aufgeschoben, bis das distale Ende der Innenhülse 50 am Stützring 86 des Schafts 44 anschlägt. Die Führungsrippen 88 oberhalb des Stützrings 86 gewährleisten dabei zusammen mit den an der Innenfläche der Innenhülse 50 vorgesehenen Längsnuten 101, dass die Innenhülse 50 und somit die gesamte in der Mitte der Figur 8 gezeigte Baugruppe drehfest am Schaft 44 festgelegt ist.

In einem weiteren Schritt wird die Befestigungsstange 48 vom proximalen Ende des Schafts 44 her in dessen Durchgangsbohrung 46 eingeführt, bis ein direkt unterhalb des Feststellgriffs 92 der Befestigungsstange 48 ausgebildeter Absatz am Abschlussring 84 des am Schaft 44 ausgebildeten Kopfabschnitts 78 anschlägt (vgl. Figur 10).

In einem weiteren Schritt wird die Griffeinheit 60 seitlich auf den Kopfabschnitt 78 aufgesetzt, wie dies in der Figur 9 gezeigt ist. Die druckfederbeaufschlagte Kugel 152 greift in der Endposition der Griffeinheit 60 in eine der Nuten 83 ein, die am Kopfabschnitt 78 ausgebildet sind, und stellt auf diese Weise eine Verliersicherung her.

In einem letzten Schritt wird nun die Rasthülse 58 wieder gelöst und durch Verdrehen in Richtung der Griffeinheit 60 bewegt. Bei der in der Figur 11 gezeigten Axialposition der Rasthülse 58 liegt der Rasthaken 144 noch nicht an der Rasthülse 58 an. Wird diese jedoch weiter gelöst und auf diese Weise zur Griffeinheit 60 hin verlagert, so greift eine schräg angeordnete Gleitfläche 156 am Rasthaken 144 an ebenfalls schräg verlaufenden Gegenflächen 158 am distalen Ende der Rasthülse 58 an. Bei fortgesetzter Axialbewegung der Rasthülse 58 wird infolge des Aufgleitens der Gleitfläche 156 auf der Gegenfläche 158 der Schwenkhebel 146 um die Schwenkachse 148 verschwenkt, wodurch der Rasthaken 144 radial nach innen bewegt wird. Bei weiter fortgesetzter Drehung der Rasthülse 58 schnappt der Rasthaken 144 schließlich hinter einer der Hinterschneidungen 124 ein, wie dies in dem Ausschnitt der Figur 12 gezeigt ist. Die auf den Schwenkhebel 146 wirkende Druckfeder 150 verhindert dabei, dass diese axiale Verrastung wieder gelöst wird.

Gleichzeitig wird auch eine Verrastung in Drehrichtung erzielt, da der Rasthaken 144 gleichzeitig in bestimmten Drehstellungen in eine der Nuten 122 eingreifen kann, die innenseitig an der Rasthülse 58 ausgebildet sind. In der Figur 13 erkennbare geneigt angeordnete Seitenflächen 160 der Nuten 122 wirken dabei mit einer ebenfalls geneigt angeordneten Seitenfläche am Rasthaken 144 zusammen, wodurch die Rasthülse 58 nur in einer Drehrichtung gedreht werden kann, während in der anderen Drehrichtung sich der Rasthaken 144 an radial verlaufenden Rastflächen 162 der Nuten 122 verfängt. Die Drehrichtung, mit der die Rasthülse 58 gedreht werden kann, ist dabei diejenige Richtung, mit der die Rasthülse 58 an die Griffeinheit 60 angenähert werden kann.

Durch diesen Rastmechanismus wird somit erreicht, dass nach Eintritt der axialen Verrastung, wie sie in der Figur 12 gezeigt ist, die Rasthülse 58 nicht mehr zurückgedreht werden kann, ohne dass die Verrastung durch manuellen Druck auf den Schwenkhebel 146 wieder gelöst wird. Ferner erzeugt das Zusammenspiel zwischen dem Rasthaken 144 und den Nuten 122 ein wiederkehrendes Klickgeräusch, wenn die Rasthülse 58 zur Griffeinheit 60 hin verdreht wird. Durch dieses Klickgeräusch wird auch akustisch wahrnehmbar, dass demnächst eine vollständige Verrastung eintreten wird. Der Rastmechanismus kann dabei z. B. so ausgelegt sein, dass etwa 3 bis 6 Klickgeräusche auftreten, bis eine vollständige Verrastung eingetreten ist.

Die Figur 14 zeigt in einem an die Figur 12 angelehnten Ausschnitt eine alternative Ausgestaltung eines Rastmechanismus. Bei diesem ist die Rasthülse 58 nicht mit Nuten 122 versehen, sondern es trägt nur eine einzige umlaufende Hinterschneidung 124 zum Rastmechanismus bei. Bei dieser Ausgestaltung bleibt die Rasthülse 58 somit auch im axial verrasteten Zustand noch drehbeweglich, und es treten im Vorfeld der Verrastung auch keine Klickgeräusche auf.

Selbstverständlich können auch zwei gleichartige Rastmechanismen vorgesehen sein. Insbesondere, wenn diese diametral einander gegenüberliegend angeordnet sind, wird dadurch ein Verkanten wirkungsvoll verhindert.

### III. Verwendung

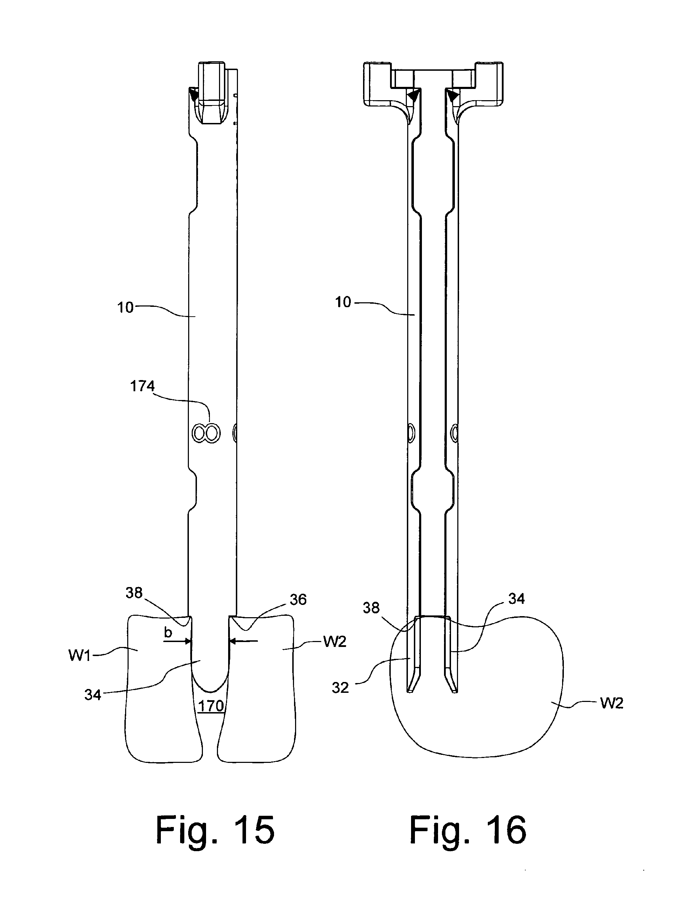

Im Folgenden wird mit Bezug auf die Figuren 16 bis 22 erläutert, wie mit Hilfe des vorstehend beschriebenen Instrumentariums das Implantat 42 in ein Bandscheibenfach eingedreht wird.

In einem ersten Schritt wird ein Zugang zu dem Bandscheibenfach freigelegt. Dabei kann es sich beispielsweise um einen posterior-lateralen Zugang handeln. In den meisten Fällen wird dann die Bandscheibe ganz oder teilweise entfernt. Die das Bandscheibenfach begrenzenden Wirbel bewegen sich dabei im Allgemeinen aufeinander zu, wodurch sich die Höhe des Bandscheibenfachs zunächst verringert.

Um das Bandscheibenfach wieder zu distrahieren, kann ein Satz mehrerer Distraktoren verwendet werden, wie dies in der oben bereits erwähnten unveröffentlichten deutschen Patentanmeldung "System zum Distrahieren eines Bandscheibenfachs" der Anmelderin beschrieben ist. Das Bandscheibenfach wird dabei sukzessive durch Verwendung von zunehmend größeren Distraktoren, die jeweils einen im Querschnitt elliptischen Kopf haben, distrahiert. Die jeweils erhaltene Distraktion wird in jedem Schritt mit Hilfe einer passenden Führungshülse 10 aufrechterhalten. Am Ende dieses Vorgangs wird der letzte Distraktor herausgezogen, so dass die Distraktion ausschließlich von einer der Führungshülsen 10 aufrechterhalten wird. Die Größe der zuletzt eingesetzten Führungshülse 10 hängt dabei u. a. von der Größe des Bandscheibenfachs und den Abmessungen des Implantats 42 ab, das in das Bandscheibenfach eingeführt werden soll.

In den Figuren 15 und 16 ist dieser Zustand in einer Seitenansicht bzw. einer Vorderansicht der Führungshülse 10 gezeigt. In der Seitenansicht der Figur 15 ist erkennbar, dass die beiden Distanzzungen 32, 34 in das mit 170 bezeichnete Bandscheibenfach hineinragen. Die Distraktion des Bandscheibenfachs 170 ist dabei gleich der Breite b der Distanzzungen 32, 34. Die Schultern 36, 38, die am distalen Ende der Führungshülse 10 ausgebildet sind, stützen sich dabei an den Rändern der Wirbel W1, W2 ab, die das Bandscheibenfach 170 begrenzen.

In einem zweiten Schritt wird ein Eindrehinstrument 40 bereitgestellt, das an das einzusetzende Implantat 42 und die hierfür ausgewählte Führungshülse 10 angepasst ist. Wie eingangs bereits erwähnt wurde, ist der Schaft 44 mit den Führungsnasen 62, 64 speziell an die Geometrien unterschiedlicher Implantate 42 angepasst. Entsprechendes gilt auch für die Befestigungsstange 48, deren Durchmesser bei dem dargestellten Ausführungsbeispiel an eine Innenbohrung im Implantat 42 angepasst ist.

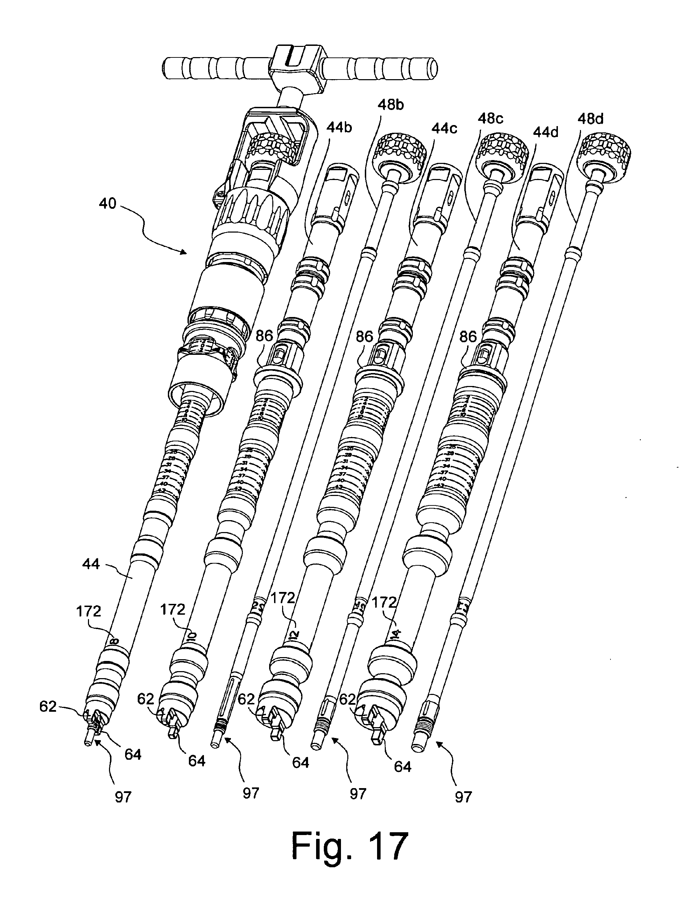

Die Figur 17 zeigt beispielhaft zusätzlich zu einem vollständig montierten Eindrehinstrument 40 mit einem Schaft 44 weitere Schäfte 44b, 44c, 44d, die zwischen dem Stützring 86 und dem distalen Ende unterschiedliche Durchmesser haben. Diese Durchmesser und insbesondere die Durchmesser der ersten Führungsringe 72 sind an Führungshülsen 10 mit unterschiedlichem Innendurchmesser angepasst. Auch die Größe der Führungsnasen 62, 64 variiert zwischen einigen der Schäfte 44, 44b, 44c, 44d.

Neben den Schäften 44b, 44c, 44d ist jeweils eine daran eingepasste Befestigungsstange 48b, 48d bzw. 48d dargestellt, die sich durch den Durchmesser des Stummels 97 und des davon getragenen Außengewindes 94 voneinander unterscheiden und nur in jeweils einen der Schäfte 44, 44b, 44c, 44d eingeführt werden können.

Im dargestellten Ausführungsbeispiel ist von den mit Zahlen 172 versehenen Schäften 44, 44b, 44c, 44d bereits derjenige Schaft 44 ausgewählt worden, der zum Implantat 42 passt und für den auch die in den Figuren 15 und 16 dargestellte und mit einer entsprechenden Zahl 174 versehene Führungshülse 10 passend ausgesucht wurde. Falls das einzusetzende Implantat 42 einen anderen Schaft 44b, 44c, 44d erfordert, so muss das links in der Figur 17 gezeigte Eindrehinstrument 40 demontiert werden. Hierzu wird zunächst die Verrastung der Rasthülse 58 durch Betätigen des Schwenkhebels 146 gelöst, die Rasthülse 58 wieder nach unten gedreht und die Griffeinheit 60 seitlich abgezogen. Dann lässt sich die Befestigungsstange 48 aus der Durchgangsbohrung 46 herausziehen und die in der Figur 8 in der Mitte gezeigte Baugruppe vom Schaft 44 abziehen. Ein solcher Umbau kann sehr schnell und sogar noch während der Operation durchgeführt werden. Dann wird die Baugruppe auf einen der anders dimensionierten Schäfte 44b, 44c oder 44d aufgeschoben und der Zusammenbau in der oben unter II geschilderten Weise fortgesetzt.

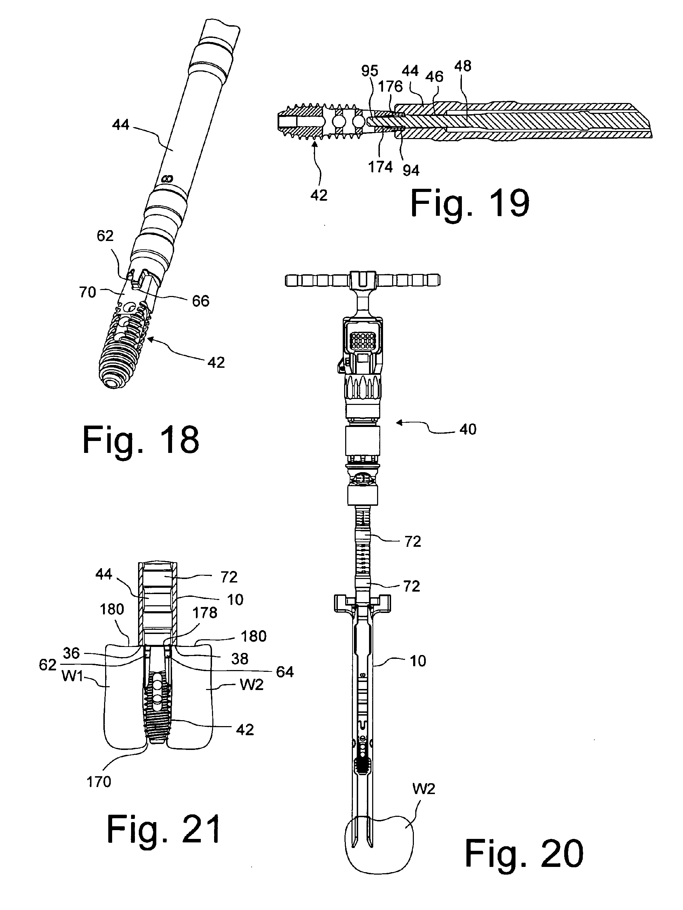

Nachdem das passende Eindrehinstrument 40 bereitgestellt wurde, wird in einem dritten Schritt das Implantat 42 am distalen Ende des Eindrehinstruments 40 befestigt. Das Implantat 42 wird hierzu, wie dies in der Figur 5 angedeutet ist, auf das distale Ende des Schafts 44 so aufgesetzt, dass die Führungsnasen 62, 64 am distalen Ende des Schafts 44 in die Führungsnuten 66 eingreifen, die am Basisabschnitt 70 des Implantats 42 ausgebildet sind. In diesem Zustand, den die perspektivische Ansicht der Figur 18 zeigt, ist das Implantat 42 drehsicher und mit vorgegebener Winkelorientierung am Schaft 44 festgelegt.

Beim Aufsetzen des Implantats 42 greift der aus dem Schaft 44 herausstehende Stummel 97 der Befestigungsstange 48 in eine axiale Bohrung 174 des Implantats 42 ein, die in dem axialen Schnitt der Figur 19 erkennbar ist. Ein oberer, zum Eindrehinstrument 40 weisender Abschnitt der axialen Bohrung 174 ist mit einem Innengewinde 176 versehen, das auf das zurückgesetzte Außengewinde 94 am Stummel 97 abgestimmt ist. Der Stummel 97 und die Anordnungen der Gewinde 94, 176 sind dabei so festgelegt, dass das Außengewinde 94 am Stummel 97 nur dann in das Innengewinde 176 des Implantats 42 eingreifen kann, wenn das Implantat 42 mit der richtigen Winkelorientierung am Schaft 44 festgelegt wurde, wie es in der Figur 18 gezeigt ist. Bei einer um 90° verdrehten Winkelorientierung würde zwar der gewindefreie vordere Abschnitt des Stummels 97 ein Stück weit in die axiale Bohrung 174 des Implantats 42 eingeführt werden können, ein Eingriff der Gewinde 94, 176 wäre jedoch nicht möglich. Folglich könnte das Implantat 42 in dieser falschen Winkelorientierung nicht am Eindrehinstrument 40 befestigt werden.

In der in der Figur 18 gezeigten korrekten Winkelorientierung des Implantats 42 zum Eindrehinstrument 40 kommen die Gewinde 94, 176 jedoch zum Eingriff, so dass durch Verdrehen des Feststellgriffs 92 die Befestigungsstange 48 in das Implantat 42 eingedreht werden kann. Dadurch wird das Implantat 42 am Schaft 44 festgespannt und ist nun auch axial gesichert.

In einem vierten Schritt wird das Eindrehinstrument 40 mit dem daran befestigten Implantat 42 in die Führungshülse 10 eingeführt, wie dies in der Vorderansicht der Figur 20 gezeigt ist. Die ersten Führungsringe 72 stellen dabei eine koaxiale Ausrichtung des Schafts 44 und der Führungshülse 10 sicher, wodurch ein Verkanten vermieden wird. Vor dem Einführen kann noch mit Hilfe eines Bohrers eine Bohrung im Bandscheibenfach 170 erzeugt werden, und auch dies ggf. nur über einen Teil der gesamten Länge des Implantats 42.

Die Figur 21 zeigt in einem Ausschnitt die spätere Sollposition des Implantats 42 im Bandscheibenfach 170; der vordere Teil der Hülse 10 ist dabei entfernt, um den darin geführten Schaft 44 des Eindrehinstruments 40 erkennen zu können. Diese Sollposition ist dadurch gekennzeichnet, dass die mit 178 bezeichnete proximale Endfläche des Implantats 42 bündig mit den hinteren Rändern 180 der Wirbel W1, W2 abschließt. In dieser Sollposition befindet sich die proximale Endfläche 178 somit auf der gleichen Höhe wie die Schultern 36, 38 der Führungshülse 10, die sich an den Wirbeln W1, W2 abstützen.

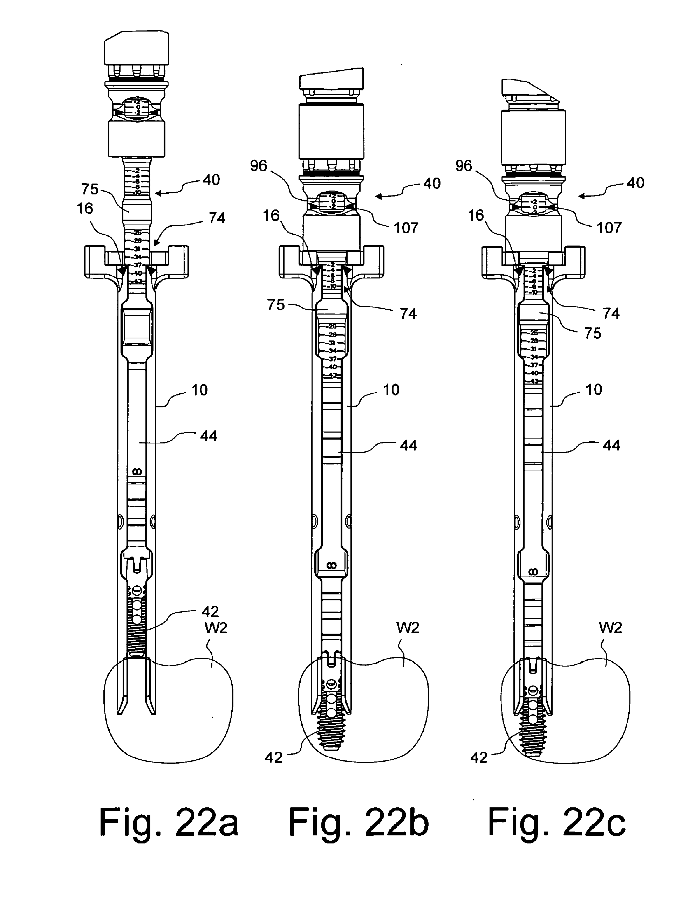

Sobald das distale Ende des Implantats 42 an den Rändern 180 der Wirbel W1, W2 anschlägt, wie dies in der Figur 22a gezeigt ist, kann in einem fünften Schritt mit dem Eindrehen des Implantats 42 in das Bandscheibenfach 170 begonnen werden. Bei der in der Figur 22a gezeigten Ausgangsstellung zeigen die Spitzen die an der Führungshülse 10 vorgesehenen Markierung 16 auf dem ersten Skalenabschnitt 74 des Schafts 44 auf einen Zahlenwert, der die Länge des Implantats 42 angibt. Der Chirurg kann sich auf diese Weise nochmals davon vergewissern, dass das am distalen Ende des Schafts 44 befestigte und nun nicht mehr sichtbare Implantat 42 die gewünschte Länge hat. Durch die anhand der Figur 21 erläuterte Festlegung der Sollposition des Implantats 42 entspricht der am ersten Skalenabschnitt 74 angezeigte Wert gleichzeitig dem Restvorschub, der noch erforderlich ist, um das Implantat 42 an seine in der Figur 21 gezeigte Sollposition zu überführen.

Zum Eindringen des Implantats 42 wird nun der Drehgriff 128 an der Griffeinheit 60 im Uhrzeigersinn gedreht. Mit Hilfe des Parallelflachs 80 am Schaft 44 überträgt sich die Drehung der Griffeinheit 60 auf den Schaft 44 und über die dort ausgebildeten Führungsnasen 62, 64 auf das Implantat 42. Dieses dreht sich nun in das Bandscheibenfach ein, wobei sich das Implantat 42 vorzugsweise sein Gewinde selbst dreht.

Beim Eindrehen des Implantats 42 in das Bandscheibenfach 70 rückt das gesamte Eindrehinstrument 40 kontinuierlich in axialer Richtung nach. Am ersten Skalenabschnitt 74 kann dabei stets abgelesen werden, wie weit das Implantat 42 in von seiner axialen Sollposition entfernt ist. In dem Bereich 75 ist bei dem dargestellten Ausführungsbeispiel der erste Skalenabschnitt unterbrochen, da der erforderliche Restvorschub noch relativ groß ist und ein fortwährendes Ablesen des Restvorschubs den Chirurgen möglicherweise nur ablenken würde. Der erste Skalenabschnitt 74 wird erst wieder fortgesetzt, wenn nur noch -10 mm Restvorschub verbleiben.

Sobald nur noch -2 mm Restvorschub verbleiben, schlägt der distale Rand der Anzeigehülse 52 am Steg 27 an, der sich zwischen den Vorsprüngen 20, 22 der Führungshülse 10 erstreckt. Dieser Zustand ist in der Figur 22b gezeigt. Der zweite Skalenabschnitt 96 auf der Innenhülse 50 ist dabei so an den ersten Skalenabschnitt 74 angepasst, dass in dieser axialen Position der am ersten Skalenabschnitt 74 ablesbare Restvorschub von -2 mm auch durch die Sichtfenster 106 hindurch am zweiten Skalenabschnitt 96 auf der Höhe der Markierungen 107 der Anzeigehülse 52 ablesbar ist. Der Anschlag der Anzeigehülse 52 an der Führungshülse 10 und die dann einsetzende axiale Verschiebung der Anzeigehülse 52 weist den Chirurgen optisch gut wahrnehmbar darauf hin, dass sich das Implantat 42 nun seiner Sollposition nähert und der Eindrehvorgang nun mit besonderer Aufmerksamkeit fortgesetzt werden muss, damit das Implantat 42 nicht versehentlich zu weit in das Bandscheibenfach 170 eingedreht wird.

Die Figur 22c zeigt die Anordnung des Eindrehinstruments 40 und der Führungshülse 10 zu dem Zeitpunkt, bei dem das Implantat 42 seine in der Figur 21 gezeigte Sollposition erreicht hat. Im Sichtfenster 106 der Anzeigenhülse 52 ist dies für den Chirurgen dadurch erkennbar, dass der Restvorschub genau 0 mm beträgt. Der Chirurg kann nun das Erreichen der korrekten Sollposition zusätzlich durch bildgebende Verfahren überprüfen.

Kommt er zu dem Ergebnis, dass das Implantat 42 optimal im Bandscheibenfach 170 positioniert ist, so kann der Chirurg noch die gewünschte winkelmäßige Orientierung des Implantats 42 im Bandscheibenfach 170 festlegen. Da das Implantat 42 im Bereich des Basisabschnitts 70 einen rechteckigen Querschnitt hat, kann ein und dasselbe Implantat 42 zwei unterschiedliche Winkel zwischen den Wirbeln W1, W2 festlegen, nämlich je nach dem, in welcher Winkelorientierung sich das Implantat 42 im Bandscheibenfach 170 befindet. Der Chirurg erkennt die Winkelorientierung des Implantats 42 am Verbindungsabschnitt 130 der Griffeinheit 60, die genauso wie der Basisabschnitt 70 des Implantats 42 einen rechteckigen Querschnitt hat. Um zwischen den beiden möglichen Winkelorientierungen der Wirbel W1, W2 hin und her zu wechseln, muss der Chirurg lediglich den Drehgriff 128 der Griffeinheit 60 zwischen zwei um 90° zueinander verdrehten Winkelorientierungen hin und her bewegen. Wegen der relativ geringen Steigung des Außengewindes 43 des Implantats 42 verändert sich dessen axiale Lage im Bandscheibenfach 170 dabei kaum.

Ist auch die Winkelorientierung in der gewünschten Weise festgelegt, so löst der Chirurg in einem sechsten Schritt die Befestigung des Implantats 42 am Schaft 44, indem er durch Betätigung des Feststellgriffs 92 die Schraubverbindung zwischen der Befestigungsstange 48 und dem Implantat 42 löst. Das Eindrehinstrument 40 kann nun aus der Führungshülse 10 herausgezogen werden, während das Implantat 42 in seiner Sollposition im Bandscheibenfach 170 bleibt.

Stellt der Chirurg bei der Überprüfung mittels bildgebender Verfahren fest, dass noch geringfügige Korrekturen erforderlich sind, so kann er diese noch vor dem Lösen der Verbindung zum Implantat 42 vornehmen. Der zweite Skalenabschnitt 96 im Sichtfenster 106 der Anzeigehülse 52 zeigt ihm dabei stets an, wie weit er sich von der Sollposition (d. h. Restvorschub = 0 mm) in beide Richtungen entfernt.

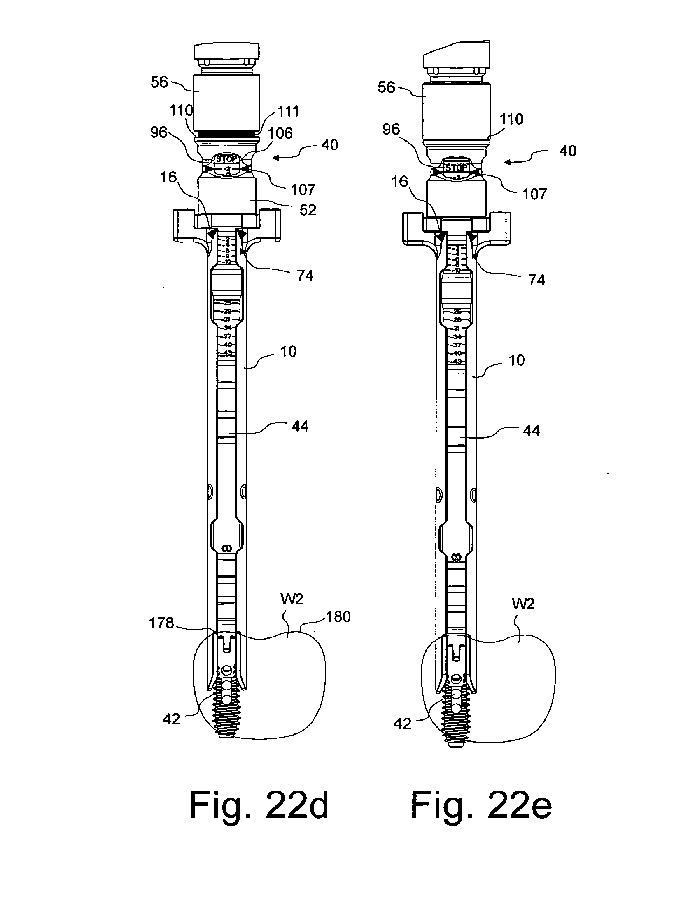

In der Figur 22d ist beispielhaft ein Zustand gezeigt, bei dem der Chirurg das Implantat 42 noch über die Sollposition hinaus in das Bandscheibenfach 170 eingedreht hat. In dieser Position befindet sich die proximale Endfläche 178 des Implantats 42 nicht mehr auf der Höhe der hinteren Ränder 180 der Wirbel W1, W2, wie dies in der Figur 22d erkennbar ist. Im Sichtfenster 106 der Anzeigehülse 52 zeigt der zweite Skalenabschnitt 77 deswegen durch einen Wert +2 mm an, dass das Implantat sich bereits 2 mm über seiner Sollposition hinaus im Bandscheibenfach 170 befindet. Ferner erscheint bei dieser axialen Position des Implantats 42 im Sichtfenster 106 der Anzeigehülse 52 der Aufdruck STOP, wodurch dem Chirurgen angezeigt wird, dass der Eindrehvorgang nun nicht weiter fortgesetzt werden darf, um eine Beschädigung empfindlichen Gewebes durch das Implantat 42 zu vermeiden. In dieser Stellung befindet sich die oberhalb des Absatzes 112 an der Anzeigehülse 52 ausgebildete Ringnut 111 genau unterhalb der Anschlagshülse 56, wodurch eine Art Helligkeitswechsel erzielt wird, der den Chirurgen zusätzlich darauf aufmerksam macht, dass die axiale Sollposition des Implantats 42 bereits überschritten wurde und die Anzeigehülse 52 kurz davor ist, an der Anschlagshülse 58 anzuschlagen.

Setzt der Chirurg dennoch den Eindrehvorgang fort, so schlägt die Schulter 110 der Anzeigehülse 52 schließlich an der Anschlagshülse 56 an, so dass der axial gegenüber der Anschlagshülse 56 festgelegte Schaft 44 nicht weiter abgesenkt werden kann. Gleichzeitig erscheint im Sichtfenster 106 der Anzeigehülse 52 die Beschriftung STOP zwischen den Markierungen 107. Dieser Zustand ist in der Figur 22e gezeigt

Diese durch einen Anschlag bewirkte Begrenzung der axialen Eindringtiefe wird erreicht, ohne dass der Chirurg im Vorfeld einen verstellbaren Anschlag am Schaft 44 justieren und feststellen müsste. Da solche Verstellvorgänge fehlerträchtig sind, kann sich der Chirurg nicht unbegrenzt auf die richtige Einstellung eines solchen verstellbaren Anschlags verlassen. Beim erfindungsgemäßen Eindrehinstrument 40 hingegen sind keine solchen verstellbaren Anschläge erforderlich. Der Chirurg könnte deswegen sogar ohne jegliche Überprüfung durch bildgebende Verfahren und die damit einhergehende Strahlenbelastung das Implantat 42 sicher in seine Sollposition eindrehen und dort noch innerhalb gewisser Grenzen axial justieren. Erreicht wird bei dem erfindungsgemäßen Instrumentarium der zuverlässige und die Eindringtiefe begrenzende Anschlag durch das Zusammenwirken der Führungshülsen 10 und des speziell daran angepassten Eindrehinstruments 40.

## Patentansprüche

1. Instrumentarium zum Eindrehen eines mit einem Außengewinde (43) versehenen Implantats (42) in ein Bandscheibenfach (170), mit:
a) einer Führungshülse (10), die
- ein distales Ende hat und sich während des Eindrehvorgangs an Wirbeln (W1, W2) abstützt, die das Bandscheibenfach (170) begrenzen, und
- ein proximales Ende hat, das während des Eindrehvorgangs zum Chirurgen weist, und mit
b) einem Eindrehinstrument (40), das einen Schaft (44) hat, der in die Führungshülse (10) einführbar ist, wobei der Schaft (44)
- ein distales Ende hat, an dem das Implantat (42) drehfest festlegbar ist, und
- ein proximales Ende hat, an dem ein Drehgriff (128, 60) drehfest befestigt ist und das während des Eindrehvorgangs über das proximale Ende der Führungshülse (10) hinausragt,
**dadurch gekennzeichnet, dass**
das Eindrehinstrument eine Restvorschubanzeige (74, 96, 52, 106, 107) aufweist, die dem Chirurgen während des Eindrehvorgangs quantitativ anzeigt, wie weit das Implantat (42) in axialer Richtung von einer axialen Sollposition entfernt ist, die relativ zu dem Ort festgelegt ist, an dem sich die Führungshülse (10) an den Wirbeln (W1, W2) abstützt.

2. Instrumentarium nach Anspruch 1, **dadurch gekennzeichnet, dass** das die Restvorschubanzeige (74, 96, 52, 106, 107) aufweist derart ausgebildet ist, dass sie auch ein Überschreiten der Sollposition in axialer Richtung quantitativ anzeigt.

3. Instrumentarium nach Anspruch 1 oder 2, **dadurch gekennzeichnet, dass** die Restvorschubanzeige eine Skala (74, 96) umfasst, die auf den Schaft (44) oder auf ein daran axial festgelegtes Bauteil (50) aufgebracht ist und mit der Führungshülse (10) zusammenwirkt.

4. Instrumentarium nach Anspruch 3, **dadurch gekennzeichnet, dass** das proximale Ende der Führungshülse (10) mit einer Markierung (16, 18) versehen ist, die auf die Skala (74) gerichtet ist, wenn das Eindrehinstrument in die Führungshülse (10) eingeführt ist.

5. Instrumentarium nach Anspruch 3 oder 4, **dadurch gekennzeichnet, dass** das Eindrehinstrument (40) eine relativ zu dem Schaft (44) axialbeweglich geführte Anzeigehülse (52) aufweist, die während des Einführens des Eindrehinstruments (40) in die Führungshülse (10) oder während des Eindrehvorgangs an dem proximalen Ende der Führungshülse (10) anschlägt und mit einem Sichtfenster (106) versehen ist, durch das hindurch die Skala (96) erkennbar ist.

6. Instrumentarium nach Anspruch 5, **dadurch gekennzeichnet, dass** der Rand des Sichtfensters (106) mit einer Markierung (107) versehen ist, die auf die Skala (96) gerichtet ist.

7. Instrumentarium nach Anspruch 5 oder 6, **dadurch gekennzeichnet, dass** das Eindrehinstrument (40) ein elastisches Element (54) aufweist, das die Anzeigehülse (52) in axialer Richtung derart vorspannt, dass die Anzeigehülse (52) während des Eindrehvorgangs gegen die Wirkung des elastischen Elements (54) axial relativ zu dem Schaft (44) verlagert wird.

8. Instrumentarium nach einem der Ansprüche 5 bis 7, **dadurch gekennzeichnet, dass** die Skala einen ersten Skalenabschnitt (74) aufweist, der mit der Markierung (16, 18) auf der Führungshülse (10) zusammenwirkt, und einen zweiten Skalenabschnitt (96) aufweist, der mit der Markierung (107) auf der Anzeigehülse (52) zusammenwirkt.

9. Instrumentarium nach Anspruch 8, **dadurch gekennzeichnet, dass** die beiden Skalenabschnitte (74, 96) derart aufeinander abgestimmt sind, dass dann, wenn die Anzeigehülse (52) während des Eindrehvorgangs an dem proximalen Ende der Führungshülse (10) anschlägt, nur noch der zweite Skalenabschnitt (96) den Restvorschub und ggf. ein Überschreiten der Sollposition in axialer Richtung quantitativ anzeigt.

10. Instrumentarium nach einem der vorhergehenden Ansprüche, **dadurch gekennzeichnet, dass** der Schaft (44) des Eindrehinstruments (40) eine sich über die gesamte Länge des Schafts erstreckende Durchgangsbohrung (46) hat, und dass das Eindrehinstrument (40) eine Befestigungsstange (48) aufweist, die in der Durchgangsbohrung (46) geführt und an ihrem distalen Ende mit einem Befestigungsabschnitt (97) versehen ist, mit dem das Implantat (42) in axialer Richtung an der Befestigungsstange (48) fixierbar ist.

11. Instrumentarium nach einem der vorhergehenden Ansprüche, **dadurch gekennzeichnet, dass** der dem Schaft (44) zugeordnete Drehgriff (128, 60) lösbar an dem Schaft (44) befestigt ist.

12. Instrumentarium nach Anspruch 11, **dadurch gekennzeichnet, dass** das Eindrehinstrument (40) zum Befestigen des Drehgriffs (128, 60) am Schaft einen Rastmechanismus (146, 122, 124, 58) aufweist, und dass die Verrastung des Drehgriffs (128, 60) mit dem Schaft (44) nur durch Betätigen einer federbeaufschlagten Rasttaste (146) lösbar ist.

13. Instrumentarium nach Anspruch 12, **dadurch gekennzeichnet, dass** die drehfeste Festlegung des Drehgriffs (128, 60) an dem Schaft durch Formschluss erzielt wird, und dass der Rastmechanismus den Drehgriff (128, 60) in axialer Richtung relativ zum Schaft (44) fixiert.

14. Instrumentarium nach Anspruch 13, **dadurch gekennzeichnet, dass** der Rastmechanismus eine auf den Schaft aufgeschraubte Rasthülse (58) aufweist, die im verrasteten Zustand axial fixiert und drehfest an dem Schaft (44) festgelegt ist.

15. Implantationssystem mit einem mit einem Außengewinde (43) versehenen Implantat (42), das zum Eindrehen in ein Bandscheibenfach (170) eingerichtet ist, **gekennzeichnet durch** ein Instrumentarium nach einem der vorhergehenden Ansprüche, wobei das Implantat (42) in zwei orthogonalen Richtungen, die senkrecht zu einer Längsachse des Implantats (42) verlaufen, unterschiedliche Abmessungen hat, derart, dass sich je nach Winkelorientierung des Implantats (42) im Bandscheibenfach (170) unterschiedliche Abstände und/oder Winkel zwischen den das Bandscheibenfach begrenzenden Wirbeln (W1, W2) ergeben, und wobei ein Befestigungsabschnitt (62, 64, 97) des Eindrehinstruments (40) derart eingerichtet ist, dass sich das Implantat (42) nur in genau zwei Winkelorientierungen, die sich um 180° voneinander unterscheiden, an dem Eindrehinstrument (44) drehfest festlegen lässt.

16. Implantationssystem nach Anspruch 15, **dadurch gekennzeichnet, dass** der Drehgriff (128, 60) am Eindrehinstrument (40) einen Abschnitt (130) aufweist, der in einem Schnitt senkrecht zur Längsachse des Schafts (44) zumindest im wesentlichen die Form eines Rechtecks hat, wobei die langen Seiten des Rechtecks parallel zu der Richtung verlaufen, entlang der die Abmessungen des an dem Eindrehinstrument festgelegten Implantats (42) größer sind.

17. Implantationssystem nach Anspruch 15 oder 16, **dadurch gekennzeichnet, dass** der Befestigungsabschnitt (62, 64, 97) ein Außengewinde (94) umfasst, das derart axial angeordnet ist, dass es nur in den zwei Winkelorientierungen in ein Innengewinde (176) am Implantat (42) eingreifen kann.

18. Implantationssystem nach einem der Ansprüche 15 bis 17, **dadurch gekennzeichnet, dass** das System
a) mehrere Schäfte (44, 44b, 44c, 44d) umfasst, die sich durch die Ausbildung der Befestigungsabschnitte (62, 64, 97) voneinander unterscheiden,
b) mehrere Implantate (42) unterschiedlicher Größe umfasst, die Befestigungsmittel (66) haben, mit denen sie jeweils drehfest an dem Befestigungsabschnitt (62, 64, 97) eines des Schäfte (44, 44b, 44c, 44d) festlegbar sind, wobei mindestens zwei unterschiedlich große Implantate (42) auch unterschiedlich ausgebildete Befestigungsmittel (66) haben, und dass das System ferner
c) mehrere Führungshülsen (10) umfasst, deren Innendurchmesser an die Durchmesser der Implantate (42) und der Schäfte (44) angepasst sind.
